(19) Europäisches Patentamt
European Patent Office
Office européen des brevets

(11) Publication number: **0 400 486 A2**

(12) **EUROPEAN PATENT APPLICATION**

(21) Application number: **90109907.7**

(22) Date of filing: **24.05.90**

(51) Int. Cl.[5]: **C08G 65/32, C07K 17/08, C12N 11/08, A61K 47/48**

(30) Priority: **26.05.89 JP 134226/89**

(43) Date of publication of application: **05.12.90 Bulletin 90/49**

(84) Designated Contracting States: **AT BE CH DE DK ES FR GB GR IT LI LU NL SE**

(71) Applicant: **SUMITOMO PHARMACEUTICALS COMPANY, LIMITED**
**2-8, Doshomachi 2-chome, Chuo-ku**
**Osaka-shi Osaka 541(JP)**

(72) Inventor: **Ono, Keiichi**
**3-10-4, Momoyamadai**
**Sakai-shi, Osaka 590-01(JP)**
Inventor: **Kai, Yoshiyuki**
**3-14-4, Higashishirakawadai, Suma-ku**
**Kobe-shi, Hyogo 654-01(JP)**
Inventor: **Ikeda, Yoshiharu**
**4-1-206, Ryodo-cho**
**Nishinomiya-shi, Hyogo 662(JP)**
Inventor: **Maeda, Hiroo**
**9-35, Miyano-cho**
**Takatsuki-shi, Osaka 569(JP)**

(74) Representative: **von Kreisler, Alek et al**
**Patentanwälte von Kreisler Selting Werner**
**Deichmannhaus am Hauptbahnhof**
**D-5000 Köln 1(DE)**

(54) **Polyethylene glycol derivatives, modified peptides and production thereof.**

(57) A polyethylene glycol derivative of the formula

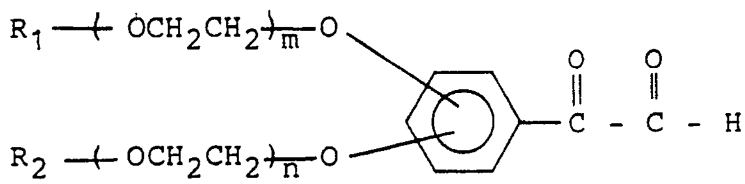

wherein $R_1$ and $R_2$ are the same or different and each represents a lower alkyl and m and n are same or different and each represents an optional positive integer which renders the average molecular weight of the polyethylene glycol moiety about 1,000 to 12,000, a peptide modified by said polyethylene glycol derivative and a method for production thereof are described. The polyethylene glycol derivative (I) is capable of modifying the guanidino groups in peptides. The thereby modified peptides are extremely stable, are considerably delayed in biological clearance (i.e. the durability is extended) and exhibit their physiological activities effectively over the long period.



Xerox Copy Centre

# POLYETHYLENE GLYCOL DERIVATIVES, MODIFIED PEPTIDES AND PRODUCTION THEREOF

## BACKGROUND OF THE INVENTION

This invention relates to polyethylene glycol derivatives which are novel and of use as peptide (particularly protein)-modifying reagent, peptides having guanidino group which are modified by said polyethylene glycol derivatives and the methods for producing them.

In recent years, with the development of the researches of proteins, a great number of peptides, in particular, physiologically active proteins having various actions have been found. Owing to the progress of genetic recombination techniques and organic synthetic methods of peptides, it has become possible to obtain these physiologically active peptides and their structurally analogous compounds in a large amount. Many of these peptides having special activity are extremely useful as pharmaceuticals.

However, it is known that the clearance of peptides which has been administered in the circulatory system is generally very fast, and therefore the improvement in durability of such peptides has been desired. Besides, since there is a risk of causing serious symptom due to the production of antibodies in the case where the peptides are obtained from different species of animals or designed by peptide•protein engineering, and they are different from those from humans in structure, the improvement of the antigenicity of said proteins is desired.

In order to use these peptides as pharmaceuticals, it is necessary to solve said problems in the aspect of their antigenicity and durability. The method of modifying the peptides chemically with macromolecular compounds is known to be extremely effective as the means by which to solve the above-mentioned problems.

Thus, polyethylene glycol derivatives have been widely used as peptide-modifying macromolecular reagents because they have excellent characteristics that they do not have immunogenicity themselves and that they do not affect the three-dimensional structures of peptides (e.g. proteins) in aqueous solutions.

In modifying the amino groups at the N-terminal or in the side-chain of the lysine residues of the peptides with derivatives having one polyethylene glycol chain, there have been known, as the general methods for activation, the activation method with polyethylene glycol triazine derivatives [Frank F. Davis et al, J. Biol. Chem., 252, 3578 - 3581 (1977)], the active ester method with N-hydroxysuccinimide [Leonardo M. et al, Tetrahedron, 40, 1581 - 1584 (1984)], the activation method with carbonyldiimidazole [Charles. O. Beecham et al, Anal. Biochem, 131, 25 - 33 (1983)], the activation method with polyethylene glycol aldehyde deriva tives [Fujino et al, Japanese Patent Application Unexamined Publication (Kokai) No. 178926/1986] and so on. In the meantime, as the method for activation with derivatives having two polyethylene glycol chains, there has been known the activation method with polyethylene glycol triazine derivatives [Inada et al, Jpn. J. Cancer Res. (Gann), 77, 1264 -1270 (1986)].

Any of these modification methods, however, comprises modifying the amino groups at the N-terminal or in the side chain of the lysine residues of the peptides.

On the other hand, in many peptides, the amino groups at the N-terminal or those in the side chain of the lysine play an important role for the expression of physiological activities. Therefore, in the case of these peptides, modifying the amino groups with the above-mentioned activating reagents is not preferable because such modification results in the reduction of activities. Besides, in the case of the method in which the lysine residues alone are modified, there is a limitation on the sites of modification. As it is considered to be effective for the purpose of controlling properties of peptides to modify various sites other than the amino groups, extremely significant is the development of reagents modifying other functional groups.

As the reagents modifying other functional groups hitherto known, mention is made of maleimide derivatives, modifying mercapto group, amino derivatives modifying carboxyl group [Frank F. Davis et al, Japanese Patent Application Examined Publication (Kokoku) No. 23587/1981] and so on.

However, while the modification thereof is considered to be difficult unless mercapto groups exist in the form of mercapto groups on the surface of the molecules, there are actually a very few peptides having such structures. In addition, in the case of modification with amino derivatives, it is difficult to react the desired modifying reagent alone selectively since the side reactions by the amino groups in the peptides occur.

Thus, the development of a high molecular modifying reagent for the functional groups other than mentioned above is desired.

Meanwhile, as the lower molecular reagents for modifying guanidino groups in peptides, there are known phenylglyoxal (Journal of Biological Chemistry, vol. 248, 6171 (1968)), 2,3-butanedione

(Biochemistry, vol. 12, 3915 (1973)), 1,2-cyclohexanedione (Journal of Biological Chemistry, vol. 250, 557 (1975)) and so on. However, there have not been so far known any high molecular modifying reagent capable of modifying guanidino groups in peptides.

## SUMMARY OF THE INVENTION

One of the objects of the present invention is to provide derivatives having two polyethylene glycol chains, a high molecular modifying reagent, which are capable of modifying guanidino groups in peptides selectively.

Another object of this invention is to provide modified peptides which can be obtained by using said polyethylene glycol derivatives.

Furthermore, still another object is to provide methods for the production of said modified peptides.

The present inventors conducted extensive researches and studies for the purpose of attaining the above-mentioned objects to find that the below-mentioned polyethylene glycol derivatives (I) could selectively modify guanidino groups in peptides. Further researches and studies resulted in the completion of the present invention.

The first invention of the present application relates to polyethylene glycol derivatives of the formula

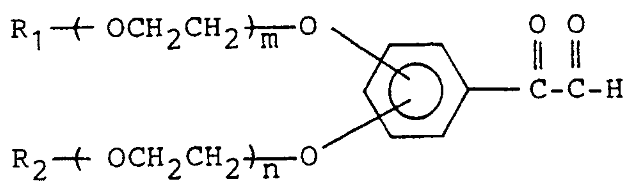

(I)

wherein $R_1$ and $R_2$ are same or different and each represents a lower alkyl and m and n represent an optional positive integer which renders the average molecular weight of the polyethylene glycol moiety about 1,000 - 12,000.

The second invention of the present application relates to modified peptides which can be obtained by reacting the polyethylene glycol derivatives (I) with peptides having guanidino group(s).

The third invention of the present invention relates to the methods for producing modified peptides which comprises reacting polyethylene glycol derivatives (I) with peptides having guanidino group(s).

## DETAILED DESCRIPTION OF THE INVENTION

In the present specification, the lower alkyl groups represented by $R_1$ and $R_2$ may be in a straight-chain or branched chain form. Preferred are, for example, lower alkyl groups having 1 to 4 carbon atoms such as methyl, ethyl, n-propyl, isopropyl, n-butyl and the like.

The polyethylene glycol derivatives (I) of the present invention can be easily produced by the following methods:

That is, by reacting a monoalkoxypolyethylene glycol of the formula

$R_1 \{ OCH_2CH_2 \}_m OH$ (II)

$R_2 \{ OCH_2CH_2 \}_n OH$ (II')

wherein $R_1$, $R_2$, m and n are of the same meanings as defined above with an appropriate activating reagent, preferably in the presence of a base, there is obtained activated compound of the formula

$R_1 \{ OCH_2CH_2 \}_m X_1$ (III)

$R_2 \{ OCH_2CH_2 \}_n X_2$ (III')

wherein $X_1$ and $X_2$ are the same or different and each represents an alkylsulfonyloxy (e.g. a lower alkylsulfonyloxy having 1 - 4 carbon atoms such as methylsulfonyloxy, ethyl sulfonyloxy or the like), an aromatic sulfonyloxy (e.g. toluenesulfonyloxy) or a halogen (chlorine, bromine, iodine, etc.) and $R_1$, $R_2$, m and n are as defined above.

As the activating reagents to be used in the reaction, mention may be made of, for example, ① alkylsulfonyl chlorides (As the alkyl moiety, preferred are the same lower alkyls as the above-mentioned alkyls. There may be mentioned, for example, methylsulfonyl chloride, ethylsulfonyl chloride and the like.)

[Ronald K. Crossland et al, J. Org. Chem. 35, 3195 (1970)], ② aromatic sulfonyl chlorides (e.g. toluenesulfonyl chloride etc.) [Vladimir C. Sekera et al, J. Amer. Chem. Soc. 55, 345 (1933)], ③ halogenated metal (e.g. sodium iodide, etc.) [G. Siewert et al., Ann., 720, 161 (1968)], ④ phosphorus pentabromide [James Cason et al, J. Org. Chem. 26, 3645 (1961)] and the like, and further, ⑤ the compounds of the formula $C(X)_4$ [wherein X represents a halogen (e.g. chlorine, bromine)] which are used in the presence of a compound of the formula $(R')_3P$ [wherein R′ represents an alkyl group (e.g. octyl), an aryl group (e.g. phenyl) or a dialkylamino group (e.g. dimethylamino)] [J. Hooz et al, Can. J. Chem. 46, 86 (1968)], and the like.

As the bases to be used in the reaction, mention may be made of pyridine, tertiary organic bases such as trialkylamine (e.g. triethylamine) and inorganic bases such as sodium hydroxide, potassium hydroxide, sodium carbonate, sodium hydride. As the reaction solvent, there can be used any per se inert solvents such as N,N-dimethylformamide, benzene, toluene, lower dialkyl ether, carbon tetrachloride, chloroform, methylene chloride, dioxane, tetrahydrofuran and the like. Some of the above-mentioned bases such as pyridine can be used as solvents themselves. The reaction temperature is usually in the range of from 0° C to 150° C.

Thereafter, by reacting the activated compound (III and/or III′) with hydroxyacetophenone in an appropriate solvent such as N,N-dimethylformamide or tetrahydrofuran in the presence of an appropriate base exemplified by an inorganic base such as potassium carbonate or sodium carbonate or an organic base such as triethylamine, tri-n-butylamine or diazabicyclo-2,2,2-undecene under heating at a temperature ranging from 60° C to 120° C, an acetophenone derivative (IV) of the formula

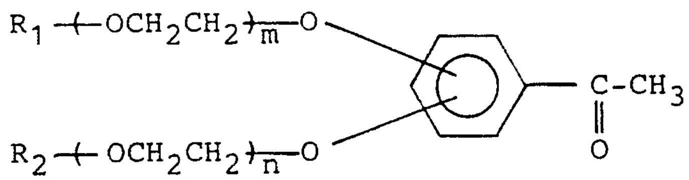

(IV)

wherein $R_1$, $R_2$, m and n are of the same meanings as mentioned above can be obtained. The acetophenone derivative (IV) can also be produced by reacting dihydroxyacetophenone with the activated compounds III or III′ with the same solvent, base and reacting temperature as mentioned above to obtain monopolyethylene glycol derivatives, followed by additional reaction with III or III′.

By oxidizing the obtained acetophenone derivative (IV) with the use of a suitable oxidizing agent such as selenium dioxide in a solvent such as N,N-dimethylformamide, or dioxane inert to the reaction at a temperature ranging from 60° C to 120° C, the objective polyethylene glycol derivative (I) can be produced.

The thus-produced polyethylene glycol derivatives (I) can be separated and purified to obtain ones having an optional purity by a per se known means.

Throughout the present specification, peptides mean compounds wherein two or more amino acids are bonded to each other by peptide linkage. Preferable are proteins and substances having structures similar to the structures of proteins. At least one of the constituent amino acids has at least one guanidino group (e.g. arginine).

As such peptides, in particular proteins, mention may be made of, for example, peptides having physiological activities such as substance P (hypothalamic hormone), corticotropin, lipotropin, melanotropin (pituitary hormone), arginine-vasopressin (neurohypophysical hormone), parathyroid hormone (thyroid hormone), thymopoietin (thymic factor), insulin, glucagon (pancreatic hormone), nerve growth factor, epidermal growth factor, insulin-like growth factor-I, insulin-like growth factor-II, human transforming growth factor, growth hormone-releasing factor, i.e. GRF, basic fibroblast growth factor (growth factor), secretin, cholecystokinin, vaso active intestinal peptide, motilin (gastrointestinal hormone), gonadotropin (chorion hormone), gonadotropic hormone-releasing hormone (gonadotropic hormone), relaxin (ovarian hormone), blood coagulation factor VIII factor and IX factor (hemophilia factor), streptokinase, fibrinolysin, deoxyribonuclease, superoxide dismutase, elastase, asparaginase (enzyme), tissue plasminogen-activator, i.e. tPA, urokinase (fibrinolysis factor), lymphokine [e.g. interleukin I and II], granulocyte macrophage•colony-stimulating factor, i.e. GM-CSF (stimulating factor), erythropoietin (erythropoietic stimulating factor), calcitonin gene related peptide (Ca-regulating hormone), atrial natriuretic peptide (diuretic hormone) and so on, and substances having structures similar to these of said peptides, which have the same physiological activities as those of said peptides.

In the above-mentioned exemplification, the uses of the peptides are described in the parentheses.

In the present invention, the peptides include any peptides that are produced by genetic engineering

methods, that are from various animals including human beings and that are produced by synthesis.

The production of the modified peptides of the present invention can be carried out by reacting polyethylene glycol derivatives (I) with peptides having guanidino group(s).

In the reaction, the polyethylene glycol derivative (I) is used preferably in about equimolar to 100 times molar amount relative to the guanidino group in the peptide. However, when it is desired to obtain peptides modified to a lower degree, the polyethylene glycol derivative (I) can be used in an amount not more than about 1 mol (e.g. 0.1 to 1 time mol) on the same basis. By adjusting the molar ratio of the polyethylene glycol derivative (I) relative to the guanidino group of the peptide having guanidino group, the reaction temperature, pH and the like, the degree of modification can be optionally selected. As the solvent to be used in the reaction, there can be used any solvent which does not prevent the reaction. Such solvents include, for example, buffer solutions such as tris hydrochloric acid buffer solution, an aqueous solution of sodium carbonate, an aqueous solution of sodium hydrogen carbonate, N-ethylmorpholine-acetic acid buffer solution, sodium maleate buffer solution and sodium acetate buffer solution. There can be added an organic solvent which does not inactivate the peptides and are inert to the reaction, exemplified by lower alcohols such as methanol, ethanol and propanol, acetonitrile, dioxane, tetrahydrofuran and the like. The pH of the reaction can be generally selected in the range of from 5.5 to 10. The pH in the neutral range to weak basic range is particularly preferable. However, in the case of peptides having unprotected α-amino group at the amino-terminal, preferred is pH 5.5 to 6.5 which is acidic. The reaction temperature may be any temperature at which the peptides are not inactivated, and is preferably in the range from 0 °C to 25 °C. The reaction can be usually conducted in the dark. The sufficient reaction time is in the range from 3 to 72 hours.

After the completion of the reaction, the reaction mixture is purified by a conventional protein-purification method such as salting-out, gel filtration, ion exchange chromatography, adsorption chromatography, affinity chromatography, ultrafiltration and preparative reversed phase HPLC, to obtain the objective modified peptides.

It is known that in the researches of phenylglyoxal as a lower molecular modifying reagent, under such severe conditions as condition that the concentration of the reagent is enhanced, the side reaction which is the elimination of the α-amino group at the amino-terminal occurs. (Biochemical Experiment Method 12, Chemical Modification of Protein (the first volume) pp 62 - 69 (Gakkai Publication Center, 1981), Journal of Biological Chemistry vol. 248, 6171 (1968)). In the modification with the use of polyethylene glycol derivatives (I) of the present invention, the possibility of the same side reaction is presumable. In the case where there is a fear of such a side reaction, advantageous are sometimes the methods which comprise protecting the amino groups in peptides having guanidino group with an appropriate protective group and then reacting the protected peptides with the polyethylene glycol derivatives (I), followed by deprotection of the amino-protective groups to produce the modified pep tides. As the amino-protective groups, usable are any protective groups that are not reactive with the polyethylene glycol derivatives (I), and can be eliminated without inactivating the peptides. Such protective groups are exemplified by succinyl group, maleyl group, 2-methylmaleyl group, 2,3-dimethylmaleyl group, exo-cis-3,6-endooxo-$\Delta^4$-tetrahydro phthaloyl group, exo-cis-3,6-endooxyhexahydro phthaloyl group, tetrafluorosuccinyl group and the like. Introduction of the protective groups can be conducted by the known methods in the art. As the protective group-introducing reagents, usable are the corresponding acid anhydrides, acyl halide, active esters and the like. In general, the corresponding acid anhydrides are often used. As the reaction solvents, usable are any solvents that do not prevent the reaction. Examples of such solvents include an aqueous solution of sodium carbonate, an aqueous solution of sodium hydrogen carbonate, N-ethylmorpholine-acetic acid buffer solution, sodium acetate buffer solution, phosphoric acid buffer solution and the like. There can be added an organic solvent which does not inactivate the peptide and is inert to the reaction. Such organic solvents are exemplified by lower alcohols such as methanol, ethanol and propanol, acetonitrile, dioxane, tetrahydrofuran and the like. The pH of the reaction can be preferably selected in the range from 6 to 10. The pH in the neutral range to weak basic range is particularly preferable. The reaction temperature may be any temperature at which the peptide is not inactivated, and is preferably in the range from -5 °C to 25 °C. The sufficient reaction time is from 30 minutes to 36 hours.

After the completion of the reaction, the reaction mixture is purified by a conventional peptide and protein-purification method such as salting-out, gel filtration, ion exchange chromatography, adsorption chromatography, affinity chromatography, ultrafiltration, preparative reversed phase HPLC and the like to give the objective amino-protected peptides. The reaction mixture can, without being purified, be directly reacted with the polyethylene glycol derivative (I) to give the amino group-protected peptide modified by the polyethylene glycol derivative.

The reaction of an amino group-protected peptide having guanidino group with a polyethylene glycol

derivative (I) and the purification after the completion of the reaction can be conducted under the same reaction conditions and by the same purification method as the above-mentioned reaction conditions and purification method.

The deprotection of the amino-protective groups can be conducted by maintaining the reaction mixture in an acidic condition. The pH is preferably selected in the range from 1 to 6. As the methods for maintaining acidic conditions, any methods can be used unless they fail to inactivate the peptide. Such methods include, for example, the methods with the use of acids (organic acids such as acetic acid trifluoroacetic acid and organic sulfonic acid; inorganic acids such as hydrochloric acid and the like) the methods with the use of acidic resins such as Dowex 50W, the methods with the use of buffer solutions such as phosphoric acid buffer solution, citric acid buffer solution, sodium acetate buffer solution and so on, and the methods comprising the above-mentioned methods in combination. The reaction is generally conducted in an aqueous solution alone. In some cases, it is preferable to add an organic solvent which does not inactivate the peptide and is inert to the reaction. Such organic solvents are exemplified by lower alcohols such as methanol, ethanol, and propanol, acetonitrile, dioxane, tetrahydrofuran and the like. As the reaction temperature, permissible is any temperature at which the peptide is not in activated. The reaction temperature is preferably in the range from 0°C to 40°C. The sufficient reaction time ranges from 5 minutes to 60 hours.

After the completion of the reaction, the reaction mixture is purified by a conventional method for protein-purification such as salting-out, gel filtration, ion exchange chromatography, adsorption chromatography, affinity chromatography, ultrafiltration, preparative reversed phase HPLC and the like to afford the objective modified peptide.

The modified peptides of the present invention can be formulated into conventional pharmaceutical preparations in a suitable conventional form such as injectable solutions for subcutaneous, intramuscular or intravenous administration. These pharmaceutical preparations can be produced by the per se known methods.

The modified peptides formulated in the form of such pharmaceutical compositions can be administered to mammals (human beings, monkeys, cows, horses, dogs, pigs etc.).

For example, in the case where the chemically modified SOD as obtained in accordance with Example 2 is administered for the treatment of acute myocardial infarction, the daily dose is usually 1 - 100 mg, which is administered in one dose to several times divided doses.

The polyethylene glycol derivatives (I) of the present invention are capable of selectively modifying the guanidino groups in peptides.

The peptides modified by the polyethylene glycol derivatives (I), as compared with the corresponding non-modified peptides, are extremely stable, are considerably delayed in biological clearance (i.e. the durability is extended) and exhibit their physiological activities effectively over the long period. Besides, the modified peptides retain the physiological activities which the non-modified peptides possess as they are. Thus, the modified peptides are very effective as the pharmaceuticals as well as the drugs for animals.

The present invention is in further detail explained by the following examples, which are not limitative to the present invention.

In the following description, each abbreviation means the following, respectively.

Asx. : aspartic acid or asparagine
Glx. : glutamic acid or glutamine
Ser. : serine,
His. : histidine,
Thr. : threonine,
Pro. : proline,
Val. : valine,
Ile. : isoleusine,
Phe. : phenylalanine,
Gly. : glycine
Arg. : arginine
Ala. : alanine
Tyr. : tyrosine
Met. : methionine
Leu. : leusine
Lys. : lysine

Example 1

(1) Production of monomethoxypolyethylene glycol tosylate

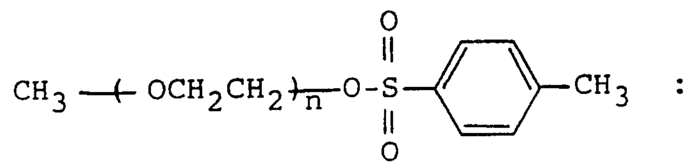

Polyethylene glycol monomethyl ether (average molecular weight 5000, 40 g) was dissolved in a mixed solvent of 160 ml of toluene and 80 ml of methylene chloride. p-Toluenesulfonyl chloride (8.0 g) was added thereto, followed by addition of 5.8 ml of triethylamine. The mixture was stirred at room temperature for 6 hours. Thereafter, p-toluenesulfonyl chloride (8.0 g) was further added, and the mixture was stirred for 10 hours. The insoluble matters were filtered off. The filtrate was concentrated under reduced pressure. The obtained residue was purified by silica gel column chromatography to give 32.4 g of the title monomethoxypolyethylene glycol tosylate (Yield 78.6%), m.p. 55 - 57° C.
$^1$H-NMR ($CDCl_3$), TMS, 90 (MHz), δ2.18 (s), δ3.38 (s), δ3.62 (s), δ7.3 (ABq)

(2) Production of monomethoxypolyethylene glycol iodide $CH_3 \leftarrow OCH_2CH_2 \rightarrow_n I$:

Monomethoxypolyethylene glycol tosylate (average molecular weight 5,000, 5 g) was dissolved in 50 ml of N,N-dimethylformamide.

Sodium iodide (766 mg) was added thereto. The mixture was stirred at 75 - 85° C for 1 hour. The insoluble matters were filtered off and the filtrate was concentrated under reduced pressure. The obtained residue was purified by silica gel column chromatography to give the title monomethoxypolyethylene glycol iodide (2.7 g), m.p. 55 - 57° C.
- Silica gel thin-film chromatography
Silica gel : Kiesel gel $60F_{254}$ (Merk Corp.)
Developing solvent : chroloform/methanol = 7/1
Rf value : 0.45

(3) Production of 3,5-bis-methoxypolyethylene glycol acetophenone

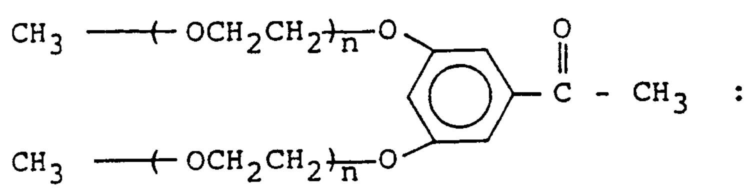

Monomethoxypolyethylene glycol-iodide (330 mg) as obtained in (2) and 3,5-dihydroxyacetophenone (5 mg) were dissolved in 2 ml of N,N-dimethylformamide. Potassium carbonate (18 mg) was added thereto, and the mixture was stirred at 90 - 95° C (bath temp.) for 1 hour.

Thereto were added monomethoxypolyethylene glycol iodide (330 mg) and potassium carbonate (18 mg), and the mixture was stirred at 90 - 95° C for 2.5 hours.

Then, monomethoxypolyethylene glycol iodide (330 mg) and potassium carbonate (18 mg) were added thereto, and the mixture was stirred at 90 - 95° C for 1.5 hours. The precipitate was filtered off. The filtrate was concentrated under reduced pressure.

The obtained residue was purified by silica gel column chromatography to give 655 mg of the title 3,5-bis-methoxypolyethylene glycol acetophenone.
- Reversed phase high performance liquid chromatography

Column: YMC-ODS, 5 μ, ∅4.6 x 250 mm
(Manufactured by Yamamura Chemicals)
Eluent : Gradient
A Solution : Water (containing 0.1% trifluoroacetic acid)
B Solution : Acetonitrile (containing 0.1% trifluoroacetic acid)
Initial concentration of B Solution : 20%
Concentration gradient : 1%/min.
Flow rate : 1 ml/min., Detection wavelength : 214 nm
Retention time : 27.4 minutes

(4) Production of 3,5-bis-methoxypolyethylene glycol phenylglyoxal

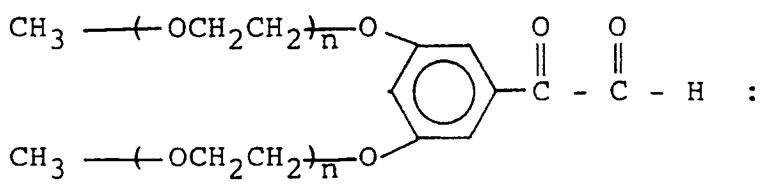

3,5-Bis-methoxypolyethylene glycol acetophenone (400 mg) as obtained in (3) was dissolved in 6 ml of 1,4 dioxane and 0.2 ml of $H_2O$. Selenium dioxide (332 mg) was added thereto, and the mixture was heated under reflux for two hours. The insoluble matters were filtered off, and the filtrate was concentrated under reduced pressure. The obtained residue was purified by gel filtration chromatography (Sephadex LH-60, ∅35 x 350 mm) to give 201 mg of 3,5-bis-methoxypolyethylene glycol phenylglyoxal, m.p. 58 - 60 °C.
- Reversed phase high performance liquid chromatography
Column: YMC-ODS, 5 μ, ∅4.6 x 250 mm
(Manufactured by Yamamura Chemicals)
Eluent : Gradient
A Solution : Water (containing 0.1% trifluoroacetic acid)
B Solution : Acetonitrile (containing 0.1% trifluoroacetic acid)
Initial concentration of B Solution : 30%
Concentration gradient : 1%/min.
Flow rate : 1 ml/min., Detection wavelength : 214 nm
Retention time : 17.4 minutes
- High performance gel filtration chromatography
Column : TSK gel G3000 SW, ∅7.5 x 600 mm
(Manufactured by Toyo Soda Corp.)
Eluent : 0.2 M NaCl (containing 5% EtOH)
Flow rate : 0.6 ml/min., Detection wavelength : 220 nm
Retention time : 25.0 minutes

## Example 2

Production of superoxide dismutase (SOD) modified by a polyethylene glycol derivative (I):

To 5.04 mg of Cu,Zn-SOD derived from human in 2.5 ml of a 0.2 M $NaHCO_3$ - 0.02 M $Na_2CO_3$ solution (pH 9.2) was added 64.5 mg of 3,5-bis-methoxypolyethylene glycol-phenylglyoxal (5 equivalent amount relative to arginine) and the mixture was kept standing in shading for 20 hours. To this mixture was added 32 mg of a modifying reagent (7.5 equivalent amount in total), and the mixture was kept standing in shading for 22 hours. After adjusting the pH of the reaction mixture to 6.4 with 2NACOH, it was purified by gel filtration on Sephacryl S-200 column (Pharmacia Corp., ∅2.6 x 94 cm). Thereafter, the mixture was subjected to desalting and concentration by ultrafiltration with the use of YM-30 membrane manufactured by Amicon Corp., USA, and thereby 1.8 ml of the solution containing the objective compound was obtained

(contained protein : 521 µg/ml).

The results of the amino acid analysis by 24 hours' treatment for acid decomposition of the objective compound with 6N hydrochloric acid-phenol at 110°C are as follows:

Asx. 31.1 (36); Glx. 26.4 (26); Ser. 16.9 (20); Gly. 47.3 (50); His. 14.2 (16); Arg. 6.14 (8); Thr. 14.2 (16); Ala. 20.4 (20); Pro. 9.05 (10); Val. 25.7 (28); Ile. 13.6 (18); Leu.* 18.0 (18); Phe. 7.93 (8); Lys. 21.3 (22)

(* means standard amino acid and the figures in parentheses are theoretical values)

- Reversed phase high performance liquid chromatography

Column: YMC-ODS, 5 µ, ⌀4.6 x 250 mm

(Manufactured by Yamamura Chemicals)

Eluent : Gradient

A Solution : Water (containing 0.1% trifluoroacetic acid)

B Solution : Acetonitrile (containing 0.1% trifluoroacetic acid)

Initial concentration of B Solution : 30%

Concentration gradient : 1%/min.

Flow rate : 1 ml/min., Detection wavelength : 214 nm

Retention time : 14.2 minutes

- High performance gel filtration chromatography

Column: TSK gel G3000 SW, ⌀7.5 x 600 mm

(Manufactured by Toyo Soda Corp.)

Eluent : 0.2 M NaCl (containing 5% EtOH)

Flow rate : 0.6 ml/min., Detection wavelength : 220 nm

Retention time : 21.1 minutes

## Example 3

Production of insulin-like growth factor-1 (IGF-1) modified by a polyethylene glycol derivative (I):

To 1.00 mg of IGF-1 in 800 µl of a 0.2 M $NaHCO_3$ - 0.02 M $Na_2CO_3$ solution (pH 9.01) was added 2.5 µl of citraconic anhydride 5 times at 5-minute intervals (12.5 µl in total) with stirring, during which addition the pH of the reaction mixture was adjusted to 8 - 9 with 10% NaOH. The reaction mixture was retained at pH 8 - 9 for 30 minutes with 1N NaOH, and the pH thereof was adjusted to 8.99. Thereafter, 122 mg of 3,5-bis-methoxypolyethylene glycol phenylglyoxal as obtained in Example 1 (15 equivalent amount relative to arginine) was added to the mixture, and the mixture was stirred at room temperature in shading for 3.5 hours. The mixture was purified by gel filtration on Sephacryl S-200 column (Pharmacia Corp., ⌀2.6 x 81 cm), and the objective fraction was subjected to desalting and concentration by ultrafiltration with the use of YM-10 membrane manufactured by Amicon Corp. to make 1.8 ml of an aqueous solution. The mixture was added AcOH to thereby adjust the pH thereof to 3.0 and the mixture was heated at 40°C for 4 hours. Then, the mixture was purified and concentrated by ultrafiltration with the use of YM-10 membrane manufactured by Amicon Corp. to give 1.8 ml of an aqueous solution containing the objective compound (contained protein : 376 µg/ml).

The results of the amino acid analysis by 24 hours' treatment for acid decomposition of the objective compound with 6N hydrochloric acid-phenol at 110°C are as follows:

Asx. 4.82 (5); Glx. 5.41 (6); Ser. 4.18 (5); Gly. 6.73 (7); Arg. 3.84 (6); Thr. 2.12 (3); Ala.* 6.00 (6); Pro. 4.50 (5); Tyr. 2.81 (3); Val. 2.34 (3); Met. 0.79 (1); Ile. 0.75 (1); Leu. 5.55 (6); Phe. 3.86 (4); Lys. 2.87 (3)

(* means standard amino acid and the figures in parentheses are theoretical values)

- Reversed phase high performance liquid chromatography

Column: YMC-ODS, 5 µ, ⌀4.6 x 250 mm

(Manufactured by Yamamura Chemicals)

Eluent : Gradient

A Solution : Water (containing 0.1% trifluoroacetic acid)

B Solution : Acetonitrile (containing 0.1% trifluoroacetic acid)

Initial concentration of B Solution : 25%

Concentration gradient : 1%/min.

Flow rate : 1 ml/min., Detection wavelength : 214 nm

Retention time : 24.39 minutes

- High performance gel filtration chromatography
Column : TSK gel G3000 SW, Ø7.5 x 600 mm
(Manufactured by Toyo Soda Corp.)
Eluent : 0.2 M NaCl (containing 5% EtOH)
Flow rate : 0.6 ml/min., Detection wavelength : 220 nm
Retention time : 22.95 minutes
Column : TSK gel G3000 PW, Ø7.5 x 600 mm
(Manufactured by Toyo Soda Corp.)
Eluent : 0.2 M NaCl
Flow rate : 0.6 ml/min., Detection wavelength : 220 nm Retention time : 19.73 minutes

## Example 4

Production of insulin-like growth factor-II(IGF-II) modified by a polyethylene glycol derivative (I):

To 1.0 mg of IGF II in 800 µl of a 0.2 M $NaHCO_3$ - 0.02 M $Na_2CO_3$ solution (pH 9.01) was added 2.5 µl of citraconic anhydride 5 times at 5-minute intervals (12.5 µl in total) with stirring, during which addition the pH of the reaction mixture was adjusted to 8 - 9 with 10% NaOH. The reaction mixture was retained for 30 minutes at pH 8 - 9 with 1N NaOH, and the pH thereof was adjusted to 8.99. To the mixture was added 107.1 mg of 3,5-bis-methoxypolyethylene glycol phenylglyoxal (10 equivalent amount relative to arginine) obtained in Example 1 and the mixture was stirred at room temperature in shading for 5 hours. Further, 107.1 mg (20 equivalent amount in total) of the modifying reagent was added to the mixture, and it was stirred at room temperature in shading for 24 hours. The reaction mixture was purified by gel filtration on Sephacryl S-200 column (Pharmacia Corp., Ø2.6 x 81 cm), and the objective fraction was subjected to desalting and concentration by ultrafiltration with the use of YM-10 membrane manufactured by Amicon Corp.. To the condensate was added a solution of 50% AcOH to thereby adjust the pH thereof to 2.98 and the mixture was stirred at 40° C for 4 hours. The mixture was subjected to desalting and concentration by ultrafiltration with the use of YM-10 membrane manufactured by Amicon Corp., to give 1 ml of an aqueous solution containing the objective compound (contained protein : 393 µg/ml).

The results of the amino acid analysis by 24 hours' treatment for acid decomposition of the objective compound with 6N hydrochloric acid-phenol at 110° C are as follows:
Asx. 2.57 (3); Glx. 6.22 (7); Ser. 6.48 (7); Gly. 4.37 (5); Arg. 6.58 (8); Thr. 3.27 (4); Ala.* 5.00 (5); Pro. 2.70 (3); Tyr. 2.53 (3); Val. 3.06 (4); Met. 0.19 (1); Ile. 0.72 (1); Leu. 4.98 (6); Phe. 3.90 (4); Lys. 1.05 (1)
(* means standard amino acid and the figures in parentheses are theoretical values)
- High performance gel filtration chromatography
Column : TSK G3000 SW, Ø7.5 x 600 mm
(Manufactured by Toyo Soda Corp.)
Eluent : 0.2 M NaCl (containing 5% EtOH)
Flow rate : 0.6 ml/min., Detection wavelength : 220 nm
Retention time : 21.0 minutes

## Example 5

Production of calcitonin gene related peptide (CGRP) modified by a polyethylene glycol derivative (I):

To 1.00 mg of CGRP derived from human in 500 µl of a 0.2 M $NaHCO_3$ - 0.02 M $Na_2CO_3$ solution (pH 9.01) was added a solution of 2.5 µl of citraconic anhydride five times at 5 minute intervals (12.5 ml in total) with stirring, during which addition the pH of the solution was kept at 8 - 9 with 10% NaOH. The mixture was kept at pH 8 - 9 for further 30 minutes with 1N NaOH, and adjusted to pH 8.99. Thereto was added 26.4 mg of 3,5-bis-methoxypolyethylene glycol phenylglyoxal (5 equivalent amount relative to arginine) and the mixture was stirred at room temperature in shading for 6 hours. To this mixture was added 26.4 mg of

the modifying reagent twice (15 equivalent amount in total), and the mixture was stirred at room temperature in shading for 60 hours. The mixture was purified by gel filtration on Sephacryl S-200 column (Pharmacia Corp., ∅2.6 x 81 cm). Thereafter, desalting and concentration of the objective fraction was conducted by ultrafiltration with the use of YM-10 membrane manufactured by Amicon Corp. Thereto was added an aqueous solution of 50% AcOH to adjust the pH thereof to 3.12. The mixture was heated at 40 °C for 4 hours, purified and concentrated by ultrafiltration with the use of YM-10 membrane manufactured by Amicon Corp. to give 1.8 µl of the solution containing the objective compound (contained protein : 164 µg/ml).

The results of the amino acid analysis by 24 hours' treatment for acid decomposition of the objective compound with 6N hydrochloric acid-phenol at 110 °C are as follows:

Asx. 3.45 (4); Ser. 2.75 (3); Gly. 4.15 (4); His. 0.77 (1); Arg. 0.87 (2); Thr. 3.68 (4); Ala.* 4.00 (4); Pro. 0.93 (1); Val. 3.70 (5); Leu. 2.66 (3); Phe. 1.69 (2); Lys. 1.90 (2)

(* means standard amino acid and the figures in parentheses are theoretical values)

- High performance gel filtration chromatography

Column: TSK gel G3000 SW, ∅7.5 x 600 mm

(Manufactured by Toyo Soda Corp.)

Eluent : 0.2 M NaCl (containing 5% EtOH)

Flow rate : 0.6 ml/min., Detection wavelength : 220 nm

Retention time : 28.04 minutes

## Example 6

Production of elastase modified by a polyethylene glycol derivative (I):

To 1.00 mg of swine elastase in 800 µl of a 0.2 M $NaHCO_3$ - 0.02 M $Na_2CO_3$ solution (pH 9.01) was added a solution of 2.5 µl of citraconic anhydride five times at 5-minute intervals (12.5 ml in total) with stirring, during which addition the pH of the solution was kept at 8 - 9 with 10% NaOH. The mixture was kept at pH 8 - 9 with 1N NaOH for further 30 minutes, and adjusted to pH 8.99. Thereto was added 46.4 mg of 3,5-bis-methoxypolyethylene glycol phenylglyoxal (10 equivalent amount relative to arginine) and the mixture was stirred at room temperature in shading for 36 hours. The mixture was purified by gel filtration on Sephacryl S-200 column (Pharmacia Corp., ∅2.6 x 81 cm). Thereafter, desalting and concentration of the objective fraction was conducted by ultrafiltration with the use of YM-30 membrane manufactured by Amicon Corp. Thereto was added an aqueous solution of 10% AcOH to adjust the pH thereof to 3.09. The mixture was heated at 40 °C for 4 hours, purified and concentrated by ultrafiltration with the use of YM-30 membrane manufactured by Amicon Corp. to give 1 ml of the solution containing the objective compound (contained protein : 389 µg/ml).

The results of the amino acid analysis by 24 hours' treatment for acid decomposition of the objective compound with 6N hydrochloric acid-phenol at 110 °C are as follows:

Asx. 21.6 (24); Glx. 17.9 (19); Ser. 20.3 (22); Gly. 24.4 (25); His. 5.95 (6); Arg. 7.93 (12); Thr. 17.5 (19); Ala.* 17.0 (17); Pro. 6.80 (7); Tyr 10.7 (11); Val. 23.2 (27); Met. 1.50 (2); Ile 8.73 (10); Leu. 15.5 (18); Phe. 2.97 (3); Lys. 3.18 (3)

(* means standard amino acid and the figures in paren theses are theoretical values)

- High performance gel filtration chromatography

Column: TSK gel G3000 SW, ∅7.5 x 600 mm

(Manufactured by Toyo Soda Corp.)

Eluent : 0.2 M NaCl (containing 5% EtOH)

Flow rate : 0.6 ml/min., Detection wavelength : 220 nm

Retention time : 23.40 minutes

## Example 7

(1) Production of

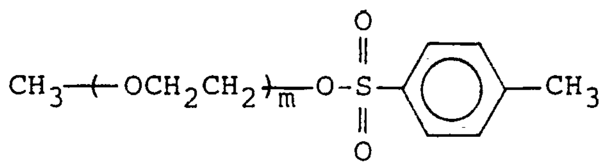

:

Polyethylene glycol monomethyl ether (average molecular weight 4,500, 100 g) was dissolved in a mixed solvent of 400 ml of toluene and 200 ml of methylene chloride.

Triethylamine (20 ml) and p-toluenesulfonyl chloride (36 g) were added thereto, followed by 5 hours' stirring at room temterature. Thereafter, triethylamine (20 ml) and p-toluenesulfonyl chloride (30 g) was further added, and the mixture was stirred for 7 hours. The insoluble matters were filtered off. The filtrate was dried to solid under reduced pressure. The obtained residue was purified by silica gel column chromatography to give 100.5 g of the title monomethoxypolyethylene glycol tosylate (Yield 97.2%).

- Reversed phase high performance liquid chromatography
Column: YMC-ODS, 5 μ, ⌀4.6 x 250 mm
(Manufactured by Yamamura Chemicals)
Eluent : Gradient
A Solution : Water (containing 0.1% trifluoroacetic acid)
B Solution : Acetonitrile (containing 0.1% trifluoroacetic acid)
Initial concentration of B Solution : 30%
Concentration gradient : 1%/min.
Flow rate : 1 ml/min., Detection wavelength : 214 nm
Retention time : 21.8 minutes

(2) Production of

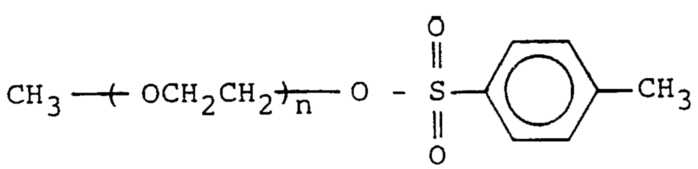

:

Polyethylene glycol monomethyl ether (average molecular weight 2,000, 50 g) was dissolved in a mixed solvent of toluene (200 ml) and methylene chloride (100 ml).

Triethylamine (10 ml) and p-toluenesulfonyl (18 g) were added thereto. The mixture was stirred at room temperature for 5 hours. Triethylamine (10 ml) and p-toluenesulfonyl chloride (15 g) were further added, and the mixture was stirred for 5 hours. The insoluble matters were filtered off, and the filtrate was dried to solid under reduced pressure. The obtained residue was purified by silica gel column chromatography to give the title monomethoxypolyethy lene glycol tosylate (40 g) (Yield : 74.3%).

- Reversed phase high performance liquid chromatography
Column: YMC-ODS, 5 μ, ⌀4.6 x 250 mm
(Manufactured by Yamamura Chemicals)
Eluent : Gradient
A Solution : Water (containing 0.1% trifluoroacetic acid)
B Solution : Acetonitrile (containing 0.1% trifluoroacetic acid)
Initial concentration of B Solution : 30%
Concentration gradient : 1%/min.
Flow rate : 1 ml/min., Detection wavelength : 214 nm
Retention time : 20.7 minutes

(3) Production of 3-hydroxy-5-monomethoxypolyethylene glycol acetophenone

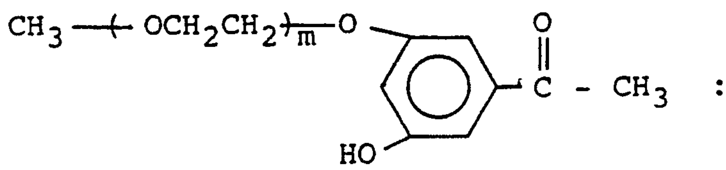

To a solution of 3,5-dihydroxyacetophenone (200 mg) in 10 ml of N,N-dimethylformamide was added potassium carbonate (364 mg, 2 equivalent amount), and the mixture was heated at 100 - 110 °C with stirring.

Thereto was dropwise added 10 ml of a N,N-dimethylformamide solution of monomethoxypolyethylene glycol tosylate (2.97 g, 0.5 equivalent amount) as obtained in (1) over a period of 1 hour, and the mixture was stirred at 100 - 110 °C for 1.5 hours. The insoluble matters were filtered off. The filtrate was dried to solid under reduced pressure. The obtained residue was purified by silica gel column chromatography and further by gel filtration (LH-60, Ø35 x 600 mm, methylene chloride - tetrahydrofuran = 6 : 1) to give 1.40 g of the title compound.

- Reversed phase high performance liquid chromatography
Column: YMC-ODS, 5 μ, Ø4.6 x 250 mm
(Manufactured by Yamamura Chemicals)
Eluent : Gradient
A Solution : Water (containing 0.1% trifluoroacetic acid)
B Solution : Acetonitrile (containing 0.1% trifluoroacetic acid)
Initial concentration of B Solution : 30%
Concentration gradient : 1%/min.
Flow rate : 1 ml/min., Detection wavelength : 214 nm
Retention time : 18.95 minutes
- High performance gel filtration chromatography
Column : TSK G3000 PW, Ø7.5 x 600 mm
(Manufactured by Toyo Soda Corp.)
Eluent : 0.2 M NaCl
Flow rate : 0.6 ml/min., Detection wavelength : 214 nm
Retention time : 25.81 minutes

(4) Production of 3 monomethoxypolyethylene glycol (average molecular weight 2,000)-5-monomethoxypolyethylene glycol (average molecular weight 4,500) acetophenone

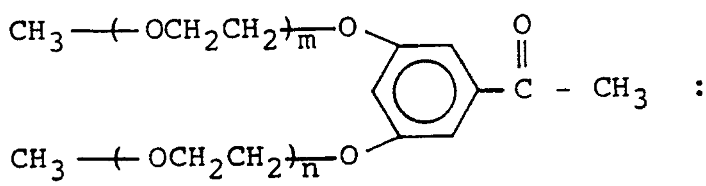

To 3-hydroxy-5-monomethoxypolyethylene glycol acetophenone (1.35 g) as obtained in (3) in an N,N-dimethylformamide solution (30 ml) were added potassium carbonate (82.8 mg) and monomethoxy polyethylene glycol tosylate (720 mg, 1.2 equivalent amount) as obtained in (2), and the mixture was stirred at 90 - 100 °C for 5.5 hours. Thereafter, monomethoxypolyethylene glycol tosylate (300 mg, 0.5 equivalent amount) was added thereto, and the mixture was stirred at the same temperature for 1.5 hours. The insoluble matters were filtered off, and the filtrate was dried to solid under reduced pressure. The obtained residue was purified by silica gel column chromatography to give 1.215 g of the title compound.

- Reversed phase high performance liquid chromatography
Column: YMC-ODS, 5 μ, Ø4.6 x 250 mm
(Manufactured by Yamamura Chemicals)
Eluent : Gradient
A Solution : Water (containing 0.1% trifluoroacetic acid)
B Solution : Acetonitrile (containing 0.1% trifluoroacetic acid)
Initial concentration of B Solution : 30%

Concentration gradient : 1%/min.
Flow rate : 1 ml/min., Detection wavelength : 214 nm
Retention time : 19.96 minutes
- High performance gel filtration chromatography
Column : TSK gel G3000 PW, ⌀7.5 x 600 mm
(Manufactured by Toyo Soda Corp.)
Eluent : 0.2 M NaCl
Flow rate : 0.6 ml/min., Detection wavelength : 220 nm
Retention time : 23.31 minutes

(5) Production of 3-monomethoxypolyethylene glycol (average molecular weight 2,000)-5-monomethoxypolyethylene glycol (average molecular weight 4,500) phenylglyoxal

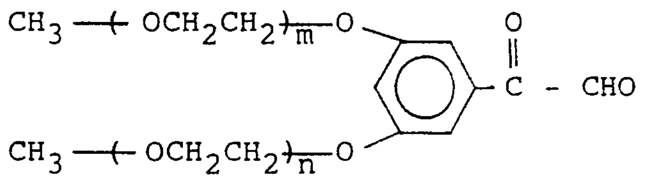 :

To 3-monomethoxypolyethylene glycol-5-monomethoxypolyethylene glycol acetophenone (600 mg) as obtained in (4) in a solution of 1,4-dioxane (9 ml) and $H_2O$ (0.3 ml) was added 614 mg of selenium dioxide (614 mg), and the mixture was heated under reflux for 5 hours. The insoluble matters were filtered off, and the filtrate was dried to solid under reduced pressure. The obtained residue was purified by silica gel column chromatography to give 400 mg of the title compound.
- Reversed phase high performance liquid chromatography
Column: YMC-ODS, 5 μ, ⌀4.6 x 250 mm
(Manufactured by Yamamura Chemicals)
Eluent : Gradient
A Solution : Water (containing 0.1% trifluoroacetic acid)
B Solution : Acetonitrile (containing 0.1% trifluoroacetic acid)
Initial concentration of B Solution : 30%
Concentration gradient : 1% min.
Flow rate : 1 ml min., Detection wavelength : 214 nm
Retention time : 18.51 minutes
- High performance gel filtration chromatography
Column : TSK gel G3000 PW, ⌀7.5 x 600 mm
(Manufactured by Toyo Soda Corp.)
Eluent : 0.2 M NaCl
Flow rate : 0.6 ml/min., Detection wavelength : 220 nm
Retention time : 23.41 minutes

## Example 8

Production of superoxide dismutase (SOD) modified by a polyethylene glycol derivative (I):

To 5.0 mg of Cu,Zn-SOD derived from human in 2 ml of a 0.1 M borate buffer solution (pH 8.21) was added a solution of 164 mg of 3-monomethoxypolyethylene glycol-5-monomethoxypolyethylene glycol phenylglyoxal (20 equivalent amount relative to arginine), and the mixture was kept standing in shading for 30 hours. After adjusting the pH of the reaction mixture to 6 with 10% AcOH, the mixture was purified by gel filtration on Sephacryl S-200 (Pharmacia Corp., ⌀2.6 x 81 cm). Thereafter, the objective fraction was subjected to desalting and concentration by ultrafiltration with the use of YM-30 membrane manufactured by

Amicon Corp., and thereby 1.8 ml of the solution containing the objective compound was obtained (contained protein : 168 µg/ml).

The results of the amino acid analysis by 24 hours' treatment for acid decomposition of the objective compound with 6N hydrochloric acid-phenol at 110 °C are as follows:

Asx. 37.6 (36); Glx. 24.8 (26); Ser. 20.8 (20); Gly. 48.0 (50); His. 18.7 (16); Arg. 6.03 (8); Thr. 15.1 (16); Ala.* 20.0 (20); Pro. 10.2 (10); Val. 22.4 (28); Ile. 10.8 (18); Leu. 17.0 (18); Phe. 5.80 (8); Lys. 16.4 (22)

(* means standard amino acid and the figures in parentheses are theoretical values)

- High performance gel filtration chromatography

Column: TSK gel G3000 SW, Ø7.5 x 600 mm

(Manufactured by Toyo Soda Corp.)

Eluent : 0.2 M NaCl (containing 5% EtOH)

Flow rate : 0.6 ml/min., Detection wavelength : 220 nm

Retention time : 24.14 minutes

Column: TSK gel G3000 PW, Ø7.5 x 600 mm

(Manufactured by Toyo Soda Corp.)

Eluent : 0.2 M NaCl

Flow rate : 0.6 ml/min., Detection wavelength : 220 nm

Retention time : 21.12 minutes

## Claims

1. A polyethylene glycol derivative of the formula

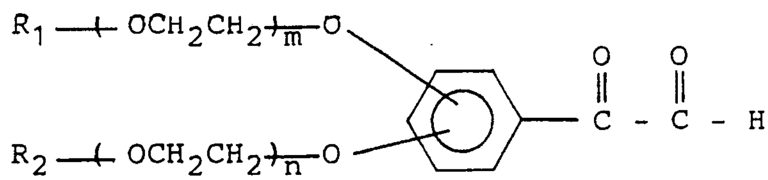

wherein $R_1$ and $R_2$ are the same or different and each represents a lower alkyl and m and n are same or different and each represents an optional positive integer which renders the average molecular weight of the polyethylene glycol moiety about 1,000 to 12,000.

2. A polyethylene glycol derivative as claimed in Claim 1 which is selected from a group consisting of 3,5-bis-methoxypolyethylene glycol phenylglyoxal and 3-monomethoxypolyethylene glycol (average molecular weight 2,000)-5-monomethoxypolyethylene glycol (average molecular weight 4,500) phenylglyoxal.

3. A modified peptide in which the guanidino group in the peptide is modified by a polyethylene glycol derivative as claimed in Claim 1.

4. A modified peptide as claimed in Claim 3 selected from among a group consisting of superoxide dismutase modified by 3,5-bis-methoxypolyethylene glycol phenylglyoxal, insulin-like growth factor-I modified by 3,5-bis-methoxy polyethylene glycol phenylglyoxal, insulin-like growth factor-II modified by 3,5-bis-methoxypolyethylene glycol phenylglyoxal, calcitonin gene related peptide modified by 3,5-bis-methoxypolyethylene glycol phenylglyoxal, elastase modified by 3,5-bis-methoxypolyethylene glycol phenylglyoxal and superoxide dismutase modified by 3-monomethoxypolyethylene glycol-5-monomethoxypolyethylene glycol phenylglyoxal.

5. A method for producing a modified peptide which comprises reacting a polyethylene glycol derivative as claimed in Claim 1 with a peptide having guanidino group.

6. A method for producing a modified peptide which comprises protecting the amino group(s) of a peptide having guanidino group and then reacting the amino-protected peptide with a polyethylene glycol derivative as claimed in Claim 1, followed by deprotection of the amino-protective group(s).